(19) Europäisches Patentamt
European Patent Office
Office européen des brevets

(11) **EP 3 474 169 A1**

(12) **EUROPEAN PATENT APPLICATION**

(43) Date of publication:
**24.04.2019 Bulletin 2019/17**

(51) Int Cl.:
**G06F 19/20** *(2011.01)*    **G06N 3/12** *(2006.01)*

(21) Application number: **17197597.2**

(22) Date of filing: **20.10.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Consejo Nacional de Investigaciones Cientificas Tecnológicas (CONICET)**
**C1425FQB Cludat Autónoma de Buenos Aires (AR)**
• **Universidad Nacional de Rosario (UNR)**
**2000 Rosario Santa Fé (AR)**

(72) Inventors:
• **Ezpeleta, Joaquín**
**2000 Rosario (AR)**
• **Krsticevic, Flavia Jorgelina**
**2000 Rosario (AR)**
• **Bulacio, Pilar Estela**
**San Nicolás de los Arroyos (AR)**
• **Tapia, Elizabeth**
**Funes (AR)**

(74) Representative: **CH Kilger Anwaltspartnerschaft mbB**
**Fasanenstrasse 29**
**10719 Berlin (DE)**

(54) **METHOD OF TAGGING NUCLEIC ACID SEQUENCES, COMPOSITION AND USE THEREOF**

(57)    A first aspect of the present invention relates to a method of tagging a nucleic acid molecule with a predetermined ID number, the method comprising: (a) attaching to said nucleic acid molecule a nucleic acid tag to form a tagged nucleic acid molecule, wherein said nucleic acid tag comprises one or more nucleic acid tag sub-units each consisting of groups of at least two nucleotides, wherein said nucleic acid tag is attributed said ID number by performing the following steps: (i) converting each nucleotide in said nucleic acid tag sub-units into a number ranging from 0 to 3, thereby creating numerical tag sub-units, wherein the distribution and content of the nucleotides in the nucleic acid tag sub-units has been configured to allow a finite number of numerical tag sub-units, (ii) attributing to each of said numerical tag sub-units a predetermined numerical tag element, thereby creating a numerical tag, (iii) linearly decoding said numerical tag, thereby creating said ID number.

A second aspect of the present invention relates to a method for multiplex sequencing and/or demultiplexing, the method comprising the steps of: (a) multiplexing amplification of tagged nucleic acid molecules obtained according to the method of the first aspect of the present invention to generate a plurality of said tagged nucleic acid molecules, (b) pooling and parallel sequencing said plurality of tagged nucleic acid molecules, thereby generating a plurality of sequence reads, and (c) demultiplexing said plurality of sequence reads, wherein each of said sequence reads is attributed to a sample.

A third aspect of the present invention is directed to a tagged nucleic acid molecule construed according to the method of the first aspect of the present invention and its use in a method for multiplex sequencing and/or demultiplexing.

A fourth aspect of the present invention relates to an apparatus configured for multiplex sequencing demultiplexing, the apparatus comprising: tools for designing nucleic acid tags according to the method described in the first aspect of the present invention, tools for pooling and multiplexing a plurality of tagged nucleic acid sequences, a sequence demultiplexer, additional tools for data reproduction and post-sequencing analysis.

A fifth aspect of the present invention, there is provided a method of tagging a nucleic acid molecule with a predetermined ID number, the method comprising: (a) attaching to said nucleic acid molecule a nucleic acid tag to form a tagged nucleic acid molecule, wherein said nucleic acid tag comprises one or more nucleic acid tag sub-units each consisting of groups of at least two nucleotides, wherein said nucleic acid tag is obtained from said ID number by performing the following steps: (i) linearly encoding said ID number, thereby creating a numerical tag, wherein the numerical tag comprises a plurality of numerical tag elements; (ii) attributing to each of said numerical tag elements a numerical tag sub-unit, wherein the numerical tag sub-unit comprises a plurality

EP 3 474 169 A1

of numbers ranging from 0 to 3; (iii) attributing to each of said numerical tag sub-units a nucleic acid tag sub-unit, thereby creating said nucleic acid tag, wherein the distribution and content of the nucleotides in the nucleic acid tag sub-units has been configured to allow a finite number of numerical tag sub-units.

**Description**

**FIELD OF THE INVENTION**

The present invention relates to the fields of molecular biology and bioinformatics. More particularly, it concerns a method of tagging nucleic acid sequences. The present invention also encompasses a tagged nucleic acid composition and use thereof in a method for multiplex sequencing and/or demultiplexing.

**BACKGROUND OF THE INVENTION**

The advent of the next-generation sequencing (NGS) technologies has beneficially affected biological sciences such as genomics, DNA sequencing being their most representative technique. The development of more efficient sequencing platforms has provided access to a large amount of data at very high throughput and low cost, allowing to retrieve genetic information of high biological value, such as single nucleotide polymorphisms (SNPs) and structural variants.

However, NGS technologies are also characterized by some limitations. Although data production capabilities have substantially improved, an accurate identification of genomic samples remains still a challenging task. A recent study involving population-based targeted sequencing highlighted many issues related to NGS platforms, including biases in sample library generation, difficulties in mapping short reads, variation in sequence coverage depth of unique and repetitive elements, difficulties in detecting insertions and deletions (indels) with short reads (Harismendy et al. 2009, Genome Biology 10(3):R32).

In view of this scenario, the application of molecular barcodes to NGS technologies has provided a new prospective for improving NGS capacities (Parameswaran et al. 2007, Nucleic Acids Res. 35(19):e130). Molecular barcoding lays on the possibility to tag DNA fragments of different samples by using short modified oligonucleotides, known as barcodes.

DNA barcodes are artificially synthesized sequences of nucleic acids which can be arranged either as part of adapters (Meyer et al. 2007, Nucleic Acids Res. 35(15):e97) or as amplification primers (Binladen et al. 2007, PLoS ONE 2(2):e197). Further, barcodes are characterized by negligible interference with nucleic acid sequencing reactions, high resilience against sequencing errors and high multiplexing capacity.

Most importantly, the design of a barcode is affected by some limitations due to experimental and coding theoretic constraints.

Among experimental constraints, the construction of barcodes depends on the sequencing platform adopted. In this respect, it has been observed that on the Roche 454 sequencing platform the predominant type of errors is represented by indels (Dohm et al. 2008, Nucleic Acids Research 36(16):e105), while on the Illumina sequencing platform the most frequent errors are substitutions (Minoche et al. 2011, Genome Biology 12:R112). Additional experimental constraints to be considered are the number of guanine (G) and cytosine (C) (GC-content) and presence of homopolymers in the target nucleic acid sequence. In fact, it appears that GC-rich parts tend to form secondary structures causing enzyme stalling or drop-off at GC-rich sequences, while homopolymers cause enzyme slipping prompting to indel errors.

Regarding coding theoretic constraints, barcodes are generally constructed according to metrics imposed by the type of sequencing errors. Barcodes must ensure a minimum pairwise distance $d_{min}$ that guarantees the unambiguous correction of $\left[\frac{d_{min}-1}{2}\right]$ sequencing errors. While the Hamming distance metric is used for substitution errors, the Levenshtein distance is used for substitution, insertion and deletion errors. Regarding the accomplishment of the $d$ constraint, barcodes can be designed by random or systematic, i.e., coding theory-based, methods. In the former case, random candidate barcode sequences are generated and exhaustive pairwise distance evaluations are performed to ensure the d constraint. While both approaches are appropriate for low sequencing noise scenarios requiring low d values, that can easily be accomplished on short barcode designs, things change as sequencing noise grows. In high sequencing noise scenarios, high d settings that can be only accomplished on long barcode sequences. In such sequencing scenarios, the random approach turns to be definitely inappropriate: while the search space scales exponentially with the length of barcodes, pairwise distance valuations between them scale linearly, in the case of the Hamming distance, or quadratically, in the case of the Levenshtein alternative. In these cases, systematic barcode designs, i.e., based on the Theory of Error Correcting Codes, are more appropriate (Tapia et al. 2015, PLoS ONE 10(10):e0140459).

Recently, a new class of barcodes have been proposed by Kracht and Schober, called watermark barcodes (Kracht and Schober 2015, BMC Bioinformatics 16:50) by adapting the concept of watermark error-correcting codes firstly developed by Davey and MacKay (Davey and MacKay 1998 Communications Letters IEEE 2(6):165-167) to deal with synchronization and substitution errors in digital communications. In this adaptation, synchronization errors are modeled as random insertion and deletion of symbols, which can be associated with sequencing indels.

Although the watermark barcode approach of Kracht and Schober appeared to be promising to deal with indels and substitution errors, it has been observed that the use of such barcodes still produce a relevant sample misassignment rate of approximately 5% in single-molecule real-time (SMRT) sequencing.

More recently, Ezpeleta proposed an alternative watermark barcoding construction based on the serial concatenation of short low-density parity check (LDPC) outer code and a non-sparse inner code (Ezpeleta et al. 2017, Bioinformatics, 33(6):807-813). The modification introduced by Ezpeleta to the design of the inner code and the decoding algorithm improved the multiplexing capacity of the watermark barcodes, which are suitable for use in SMRT sequencing applications.

In general, the design of a barcode is based on mathematical codes like binary Hamming codes (Hamady et al. 2008, Nat. Methods 5(3):235-237), Levenshtein codes (Buschmann and Bystrykh 2013, BMC Bioinformatics 14:272), codes over quaternary alphabets (Bystrykh 2012, PLoS ONE 7(5):e36852) or BCH-codes (Krishnan et al. 2011, Electronics Letters 47(4):236-237). Such mathematical constructions are intended not only to provide means for constructing the codes, but also to decode efficiently the same.

Current barcoding systems are mostly designed with exhaustive methods. Large sets of random nucleic acid sequences of a defined size are first screened to satisfy experimental constraints imposed by the sequencing technology. Candidate barcodes are then selected to ensure a minimum pairwise distance that guarantees the unambiguous correction of sequencing errors. The choice of the distance metric is determined by the type of sequencing errors. In general, pairwise Hamming distance evaluations are required for substitution errors, while pairwise Levenshtein distance evaluations are required for substitutions, insertion and deletion errors.

However, current SMRT sequencing technologies are constrained by a critical trade-off between the cross-talk among samples and the loss of DNA reads. Cross-talk occurs among samples at demultiplexing time when DNA reads are associated with wrong samples due to the high degree of background sequencing noise.

Among single-molecule sequencing methods, nanopore sequencing is regarded as one of the most promising technologies in achieving real-time, high throughput, single molecule DNA sequencing. A nanopore-based device provides single-molecule detection and analytical capabilities that are achieved by electrophoretically driving molecules through a nanoscale pore (Branton et al. Nature 2008, Biotechnology 26:1146-1153).

Multiplex sequencing technologies with 24-nucleotide barcodes are provided for native ID sequencing chemistry with a sequencing error rate around 20%. With reference to nanopore sequencing technologies, such as MinION™, the error rate for 2D sequencing chemistry is reduced to around 10% (Quick et al. 2017, Nat. Protoc. 12(6):1261-1276; van der Helm et al. 2017, Nucleic Acids Res. 45(8):e61; Bayliss et al. 2017, Gigascience 6(3):1-6).

Despite the recent improvements and efforts in developing barcodes to provide a robust algorithm for multiplex sequencing/demultiplexing, it is evident that the current barcodes still suffer from some limitations, such as capacity in assigning reads correctly to a sequence in presence of indel and substitutions errors. Moreover, some barcodes are not suitable to be used for all NGS technologies, wherein a high capacity of elaborating and providing data in real-time is required.

It is therefore highly demanded to provide a new method for construction of a barcode capable to deal with experimental constraints in a fast and reliable way and thus, being suitable for use in different sequencing platforms.

## SUMMARY OF THE INVENTION

A first aspect of the present invention relates to a method of tagging a nucleic acid molecule with a predetermined ID number, the method comprising: (a) attaching to said nucleic acid molecule a nucleic acid tag to form a tagged nucleic acid molecule, wherein said nucleic acid tag comprises one or more nucleic acid tag sub-units each consisting of groups of at least two nucleotides, wherein said nucleic acid tag is attributed said ID number by performing the following steps: (i) converting each nucleotide in said nucleic acid tag sub-units into a number ranging from 0 to 3, thereby creating numerical tag sub-units, wherein the distribution and content of the nucleotides in the nucleic acid tag sub-units has been configured to allow a finite number of numerical tag sub-units, (ii) attributing to each of said numerical tag sub-units a predetermined numerical tag element, thereby creating a numerical tag, (iii) linearly decoding said numerical tag, thereby creating said ID number.

A second aspect of the present invention relates to a method for multiplex sequencing and/or demultiplexing, the method comprising the steps of: (a) multiplexing amplification of tagged nucleic acid molecules obtained according to the method of the first aspect of the present invention to generate a plurality of said tagged nucleic acid molecules, (b) pooling and parallel sequencing said plurality of tagged nucleic acid molecules, thereby generating sequence reads, and (c) demultiplexing said sequence reads, wherein each of said sequence reads is attributed to a sample.

A third aspect of the present invention is directed to a tagged nucleic acid molecule construed according to the method described in the first aspect of the present invention and its use in a method for multiplex sequencing and/or demultiplexing.

A fourth aspect of the present invention relates to an apparatus configured for multiplex sequencing demultiplexing, the apparatus comprising: tools for designing nucleic acid tags according to the method described in the first aspect of the present invention, tools for pooling and multiplexing a plurality of tagged nucleic acid sequences, a sequence demultiplexer, and additional tools for data reproduction and post-sequencing analysis.

A fifth aspect of the present invention relates to a method of tagging a nucleic acid molecule with a predetermined ID number, the method comprising: (a) attaching to said nucleic acid molecule a nucleic acid tag to form a tagged nucleic acid molecule, wherein said nucleic acid tag comprises one or more nucleic acid tag sub-units each consisting of groups of at least two nucleotides, wherein said nucleic acid tag is obtained from said ID number by performing the following steps: (i) linearly encoding said ID number, thereby creating a numerical tag, wherein the numerical tag comprises a plurality of numerical tag elements, (ii) attributing to each of said numerical tag elements a numerical tag sub-unit, wherein the numerical tag sub-unit comprises a plurality of numbers ranging from 0 to 3, (iii) attributing to each of said numerical tag sub-units a nucleic acid tag sub-unit, thereby creating said nucleic acid tag, wherein the distribution and content of the nucleotides in the nucleic acid tag sub-units has been configured to allow a finite number of numerical tag sub-units.

## DESCRIPTION OF THE DRAWINGS

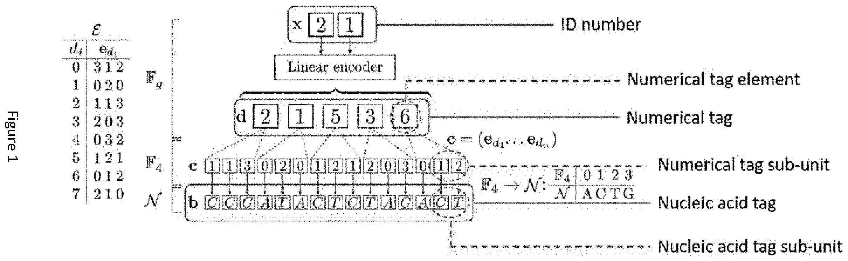

Figure 1 shows the improved barcode design algorithm according to the present invention. Said barcode design algorithm is conceived for designing a nucleic acid tag by linearly encoding an ID number into a numerical tag composed by at least three tag numbers. A numerical tag sub-unit is then associated with each tag number and, finally, a nucleic acid tag sub-unit is associated with each numerical tag sub-unit. The definition of variables disclosed in the improved barcode design algorithm can be found in Ezpeleta et al. 2017 (Bioinformatics, 33(6):807-813). As matter of example, Figure 1 illustrates one embodiment of the invention, wherein said nucleic acid tag comprises nucleic acid tag sub-units each consisting of groups of three nucleotides. However, according to a preferred embodiment, each nucleic acid tag sub-unit consists of at least two nucleotides (dashed circle).

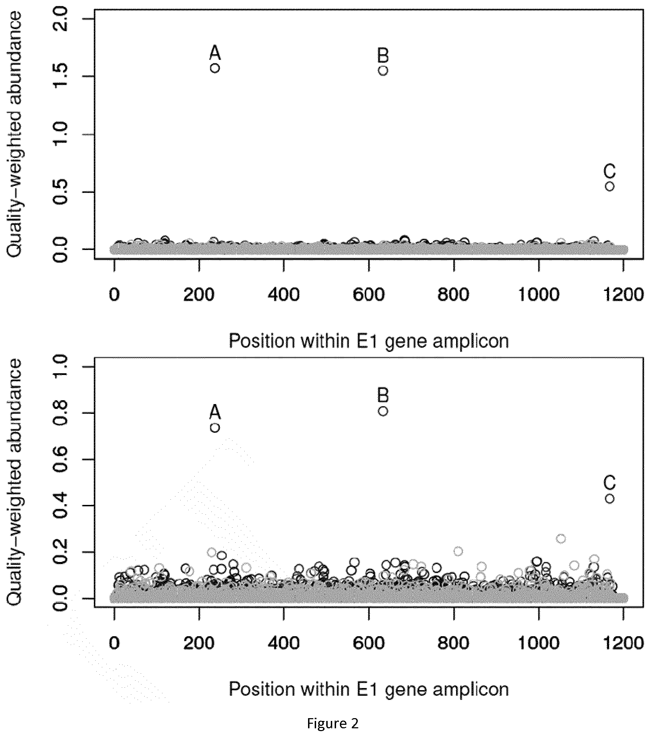

Figure 2 shows variant calling for barcode 157404 using 2D (top figure) and template (bottom figure) reads. Three candidate SNPs are clearly visible: (A) a sample-specific G->A mutation at position 238; (B) an A->C mutation at position 632 which is common to all samples; and (C) a degenerate base in the reverse primer, where approximately half of the sequences have C in place of a T (reference).

## DETAILED DESCRIPTION OF THE INVENTION

The present invention is conceived to solve issues affecting modern sequencing technologies at the level of decoding reads (demultiplexing). Particularly, the method disclosed herein is suitable in accurately associating sequence reads with a sample in presence of sequencing errors, such as indels and substitutions, with a negligible error rate.

The method according to the present invention is based on an improved barcode construction characterized by the absence of a watermark code (watermark sequence). A first advantage associated with this new construction is represented by the increment of decoding speed in demultiplexing samples. This aspect is particularly appreciated in real-time sequencing technologies. Surprisingly, the inventors have found that the removal of the watermark code does not affect the decoding accuracy of the barcode construction for small- to moderate length barcodes such as those used in multiplex sequencing, provided there is sufficient knowledge about barcode boundaries. Further, the inventors observed that, when no watermark sequence is used, a great reduction in complexity can be obtained by precomputing all quantities $P(r_i, x^- \rightarrow x^+|d_i = a)$ in the inner decoder equations 1, 2 and 3, reported below, which compute likelihoods $L(d_i = a)$. Precomputation of these quantities, which is the basis of the improved barcode construction disclosed herein, is possible because, in the absence of a watermark sequence, these quantities become position-independent (stationary). The reduction in complexity afforded by this precomputation is key to support the real-time and scalable decoding of the present invention.

$$L(d_i = a) = \sum_{x^-, x^+ \in X} \underbrace{P(\mathbf{r}^-, x_i = x^-)}_{F_i(x^-)}$$

$$\underbrace{P(\mathbf{r}_i, x_{i+1} = x^+|d_i = a, x_i = x^-)}_{P(\mathbf{r}_i, x^- \rightarrow x^+|d_i = a)} \underbrace{P(\mathbf{r}^+|x_{i+1} = x^+)}_{B_{i+1}(x^+)},$$

[1]

$$F_j(x_f) = \sum_{\substack{x_f^- \in X \\ a \in \mathbb{F}_q}} F_{j-1}(x_f^-) P(\mathbf{r}_{j-1}, x_f^- \to x_f | d_{j-1} = a)$$

[2]

and

$$B_k(x_b) = \sum_{\substack{x_b^+ \in X \\ a \in \mathbb{F}_q}} B_{k+1}(x_b^+) P(\mathbf{r}_k, x_b \to x_b^+ | d_k = a),$$

[3]

Definition of variables disclosed in equations [1]-[3] can be found in Ezpeleta et al. 2017 (Bioinformatics, 33(6):807-813).

A significative parameter for appreciating the improved barcode according to the present invention relates to its "complexity", which describe how the number of operations to be performed grows as a function of one or more parameters. The complexity is constant in the number of barcodes, while current alignment-based methods are linear in the number of barcodes. When comparing with watermark codes, the difference is in the computation of probabilities of the form $P(r_i, x \to x^+ | d_i = a)$. For watermark barcodes, the complexity of this computation is quadratic in the allowed drift and in the maximum number of insertions considered. For the watermarkless barcode, as defined in the present invention, it is constant in both the allowed drift and the maximum number of insertions considered.

In addition to reducing the computational complexity, the method disclosed herein is useful to design a proper matrix "E" in a more direct and interpretable way, e.g. see Figure 1, according to distance and homopolymers criteria/constraints.

In modern high-throughput sequencing techniques, a nucleic acid sample is tagged with a unique sequence, namely the nucleic acid tag or barcode, before being pooled into batches and sequenced in parallel in a single sequencing run (multiplexing). The reads obtained by the sequencing procedure can be decoded (demultiplexed) according to the numerical tag attached and therefore, assigned to the sample of origin.

According to a first aspect of the present invention, there is provided a method of tagging a nucleic acid molecule with a predetermined ID number, the method comprising:

    a. attaching to said nucleic acid molecule a nucleic acid tag to form a tagged nucleic acid molecule,

wherein said nucleic acid tag comprises one or more nucleic acid tag sub-units each consisting of groups of at least two nucleotides, wherein said nucleic acid tag is attributed said ID number by performing the following steps:

    i. converting each nucleotide in said nucleic acid tag sub-units into a number ranging from 0 to 3, thereby creating numerical tag sub-units, wherein the distribution and content of the nucleotides in the nucleic acid tag sub-units has been configured to allow a finite number of numerical tag sub-units,
    ii. attributing to each of said numerical tag sub-units a predetermined numerical tag element, thereby creating a numerical tag,
    iii. linearly decoding said numerical tag, thereby creating said ID number.

As used herein, the term "tagging" means assigning a specific and unique number to a nucleic acid molecule. According to the present invention, said number, referred also as predetermined ID number, comprises any positive integer value ranging from 0 to qk-1, wherein "q" and "k" are parameters of the outer code. The person skilled in the art is aware that the choice of "q" and "k", and therefore the numerical range, will be based on the actual tag length "l". For a given tag length "l", the stronger the error correcting power one desires, the smaller the number of available tags, i.e., the smaller the multiplexing capacity "B". In other terms, B < qk, where "qk" is the maximum attainable multiplexing capacity. As illustrated, but not limited, by the example disclosed herein, "q" is 8 and "k" is 2, meaning that the integer value defining the predetermined ID number ranges from 0 to 63. In the context of the present invention, a tag length "l" ranges from 16 to 256 nucleotides, preferably from 24 to 128 nucleotides.

The method according to the first aspect of the present invention comprises attaching to a nucleic acid molecule a nucleic acid tag to form a tagged nucleic acid molecule. As used herein, the term "attaching" refers to any chemical and/or physical interaction between a nucleic acid tag and a nucleic acid molecule that results in forming a stable complex. In this context, the term "attaching" may also be interpreted as "hybridizing" or "ligating" a nucleic acid tag to a nucleic acid molecule.

The nucleic acid tag according to the present invention can be defined following a multi-layered hierarchical structure as depicted in Figure 1. Therefore, the term "nucleic acid tag" refers to a nucleic acid sequence comprising "nucleic acid tag sub-units". Each "nucleic acid tag sub-unit" comprises a group of at least two nucleotides, wherein each nucleotide of said "nucleic acid tag sub-unit" can be associated to a number ranging from 0 to 3, referred to collectively as a "numerical tag sub-unit". Each "numerical tag sub-unit" can be further associated to a predetermined "numerical tag elements", referred to collectively as a "numerical tag". Eventually, said "numerical tag" can be linearly decoded into an "ID value".

As used herein, the terms "linearly decoding" or "linearly encoding" relate generally to a decoding or encoding method, wherein the relationship between a "numerical tag" "d", and an "ID number" "x" can be represented as the matrix product $d = x \cdot G$, where "G" is the generator matrix of a linear code, which can be obtained from the code parameters according to methods known in the art.

As used herein, the term "predetermined" refers to criteria or parameters specified within the context of algorithm design to initiate a function or to limit a function.

In the context of the present invention, nucleic acid tag sub-units are constructed as nucleic acid sequences of fixed length "u" from the four different bases. For instance, DNA bases A, C, G, T are encoded as numbers 0, 1, 2 and 3 in a quaternary alphabet. The number of all possible combinations, and therefore the size of the maximum numerical tag sub-unit set is $4^u$. After having calculated the maximal set size of numerical tag sub-unit defined in a matrix "E", a screening has been performed of said numerical tag sub-units leading to the selection of preferred strings of numbers that satisfy determined experimental and coding theoretic constraints.

The nucleic acid tag construction according to the present invention takes into consideration experimental constraints, such as indels and substitution errors. In this respect, error-correcting codes can be constructed by use Hamming and Levenshtein distances among each numerical tag sub-unit within a set of said numerical tag sub-units.

In general, the Hamming distance between a pair of numerical tag sub-unit measures the minimal number substitutions that are needed to transform one numerical tag sub-unit into the other. Said Hamming distance between numerical tag sub-units in a binary code determines its ability to detect and/or correct errors. In this respect, substitution errors can be corrected by constructing codes with a larger minimal Hamming distance between numerical tag sub-units.

According to one embodiment of the present invention, the numerical tag sub-units are selected by maximizing the minimum Hamming distance of each numerical tag sub-unit relative to the other numerical tag sub-units. In the context of the present invention, all measurements of distance refer to the Hamming distance unless otherwise specified. In order to design the best possible code, said code should have many numerical tag sub-units (codewords) and have a large distance between said numerical tag sub-units to prevent errors, but with length short enough to be easily manageable.

According to another embodiment of the present invention, each of said numerical tag elements is a number between 0 and q-1, wherein "q" is an integer greater than or equal to 8. In the context of the present invention, without limiting its scope, a preferred upper limit for "q" is 64.

It is worth noting that value of "q" is linked to computational complexity and noise, for a given tag length "l", the higher the "q", the higher the noise level than can be tolerated at the expense of computational complexity.

Barcodes are generally conceived on algorithms comprising a predetermined set of instructions for solving a specific problem in a limited number of steps. Algorithms can range from a mere succession of simple mathematical operations to more complex combination of procedures by using more involved constructions.

According to another embodiment of the present invention, the numerical tag is linearly encoded from said ID number by predetermined mathematical operations. As used herein, the term "linear encoded" refers to the matrix product $d = x \, G$, where "d" is the "numerical tag", "x" is the "ID number" and "G" is the generator matrix of a linear code, which can be obtained from the code parameters according to methods known in the art.

According to another embodiment, at least one nucleotide in the nucleic acid tag is modified with a detectable label. As used herein, the term "detectable label" refers to a molecule or a group thereof associated with a nucleotide and used to identify the tagged nucleic acid hybridized to a target nucleic acid. In the context of the present invention, each of the four nucleotides may be labeled with a different detectable label so that each nucleotide may be distinctly recognized.

According to a further embodiment, the detectable label is selected from the group consisting of fluorescent label, chromophore, radiolabel and enzyme label.

According to another embodiment of the first aspect of the present invention, the step of attaching to said nucleic acid molecule a nucleic acid tag is performed by any reaction comprising ligation of a tagged adaptor to said nucleic acid molecule and/or hybridization of a tagged oligonucleotide primer. In the context of the present invention, the nucleic acid tags may be incorporated into the nucleic acid molecule before performing a PCR reaction by ligation of a tagged adaptor to the 3'- and/or 5'-ends of said nucleic acid molecule or hybridization of a tagged oligonucleotide primer to the 3'- and/or 5'-ends of said nucleic acid molecule.

An important aspect of a barcode lays on its ability in demultiplexing a multitude of sequence reads in a fast and reliable way. Therefore, a performance of a barcode can be measured by different parameters, such as speediness in performing multiplex sequencing as well as reliability in assigning correctly reads to sequences.

From Sanger to Illumina, sequencing error rates have remained below 0.1% and, thus, demultiplexing DNA reads has remained computationally effortless. However, with the advent of single molecule and long-read sequencing technologies (3rd generation), with error rates above 10%, demultiplexing of DNA reads has become computationally challenging.

As mentioned above in the description part of the present application, the design of a barcode requires considering certain experimental constraints, i.e. the GC-content and/or the presence and length of homopolymers of the tagged nucleic acid sequence.

According to another embodiment, the nucleic acid tag is characterized by a GC-content between 35% and 65%.

According to another embodiment, the nucleic acid tag comprises a number of equal nucleotides in a row ranging from 1 to 4. As used herein the term "equal nucleotides" refers to nucleotides that are identical.

According to a second aspect of the present invention, there is provided a method for multiplex sequencing and/or demultiplexing, the method comprising the steps of:

> a. multiplexing amplification of tagged nucleic acid molecules obtained according to the method of the first aspect of the present invention to generate a plurality of said tagged nucleic acid molecules,
> b. pooling and parallel sequencing of said plurality of tagged nucleic acid molecules, thereby generating a plurality of sequence reads, and
> c. demultiplexing said plurality of sequence reads, wherein each of said sequence reads is attributed to a sample.

As used herein, the term "multiplex" refers to the step of performing simultaneously amplification or sequencing reaction of more than one target nucleic acid sequence of interest within the same reaction vessel. In the context of the present invention, the term "multiplex" refers to at least 10 or 20 different target sequences, preferably at least 100 different target sequences, more preferably at least 500 different target sequences, even more preferably at least 1000 different target sequences. Ideally, more than 5000 or 10,000 different target sequences. As used herein, the term "plurality" refers to any integer greater than 1. As used herein, the term "demultiplex" refers to the step of decoding the nucleic acid tag (barcode) in order to assign the nucleic acid sequence carrying said barcode to a sample of origin. As used herein, the term "sample" defines any biological sample obtained from a subject or a group or a population of subjects.

According to an embodiment of the second aspect of the present invention, the sequencing step is performed according to any of the method comprising: single-molecule real-time sequencing, pyrosequencing, sequencing by synthesis, sequencing by ligation, nanopore sequencing, Sanger sequencing, capillary electrophoresis sequencing.

A further advantage of the method of tagging disclosed herein is represented by its scalability in terms of design and demultiplexing time. Firstly, longer tagged nucleic acid molecule of increased multiplexing capacity or resistance to noise can be easily designed since a systematic method is provided. Secondly, demultiplexing of individual reads requires only two processing steps, one alignment/synchronization step and one decoding step, with no need to perform exhaustive searches across the complete set of barcodes.

According to another embodiment of the second aspect of the present invention, step (c) further comprises the steps of (i) alignment/synchronization of the plurality of sequence reads obtained in step (b) and (ii) decoding said plurality of sequence reads.

According to another embodiment of the second aspect of the present invention, the method is capable of demultiplexing reads having any length ranging from about 100 bp to about 100 kbp. As used herein the term "about" refers to plus or minus 10% of the referenced value.

In a further embodiment, the method for multiplex sequencing and/or demultiplexing according to the second aspect present of the present invention is characterized by a rate of sequence misassignment of about 1 in every 500 reads, preferably less than 1 in every 2500 reads, or 1 in every 500 reads. According to an alternative embodiment, the method for multiplex sequencing and/or demultiplexing according to the second aspect present of the present invention is characterized by sequencing error rates in the order of 20%, more preferably in the order of 10%.

According to another embodiment of the second aspect, wherein said nucleic acid molecule is obtained from different organisms comprising: mammals, plants, fungi, viruses and bacteria. As used herein, the term "mammals" refers to any individual of mammalian species, including primates, e.g. humans. As used herein, the terms "plants", "fungi" and "bacteria" includes reference to any living organism belonging to the respective kingdoms.

According to another embodiment of the second aspect, wherein said nucleic acid molecule is selected from the group comprising DNA, RNA, PNA and derivative thereof. As used herein, the terms "DNA", "RNA", "PNA" refers to a single linear strand of nucleotides. Further, in the context of the present invention "DNA" and "RNA" may originate from natural sources or may be of synthesis. In the context of the present invention, when "DNA" and RNA" molecules originate from natural sources, said molecules may be extracted according to methods known in the art.

According to another embodiment of the second aspect, wherein said nucleotides are selected from the group comprising adenine, cytosine, guanine, thymine, uracil and derivative thereof.

According to a third aspect of the present invention, the present invention discloses a tagged nucleic acid molecule construed according to the method described in the first aspect of the present invention.

In the context of the present invention, the terms "construed", "designed" can have same meaning and therefore used interchangeably.

In another aspect of the present invention, provided herein there is the use of a tagged nucleic acid molecule according to the third aspect in a method for multiplex sequencing and/or demultiplexing. Unless otherwise specified or indicated by the context, the terms "a" and "an" mean "one or more".

The person skilled in the art is aware of methods for multiplex sequencing and/or demultiplexing and meaning thereof. A description of said methods can be found in the literature incorporated in the background section of the present application.

According to a fourth aspect of the present invention, there is provided an apparatus configured for multiplex sequencing demultiplexing, the apparatus comprising:

- tools for designing nucleic acid tags according to the method of the first aspect of the present invention,
- tools for pooling and multiplexing a plurality of tagged nucleic acid sequences,
- a sequence demultiplexer,
- additional tools for data reproduction and post-sequencing analysis.

In the context of the present invention, terms like "tools for pooling and multiplexing a plurality of tagged nucleic acid sequences", "sequence demultiplexer" and "additional tools for data reproduction and post-sequencing analysis" refer to any instruments known in the art capable of carrying out the intended action.

According to a fifth aspect of the present invention, there is provided a method of tagging a nucleic acid molecule with a predetermined ID number, the method comprising:

(a) attaching to said nucleic acid molecule a nucleic acid tag to form a tagged nucleic acid molecule,

wherein said nucleic acid tag comprises one or more nucleic acid tag sub-units each consisting of groups of at least two nucleotides, wherein said nucleic acid tag is obtained from said ID number by performing the following steps:

(i) linearly encoding said ID number, thereby creating a numerical tag, wherein the numerical tag comprises a plurality of numerical tag elements;
(ii) attributing to each of said numerical tag elements a numerical tag sub-unit, wherein the numerical tag sub-unit comprises a plurality of numbers ranging from 0 to 3;
(iii) attributing to each of said numerical tag sub-units a nucleic acid tag sub-unit, thereby creating said nucleic acid tag,

wherein the distribution and content of the nucleotides in the nucleic acid tag sub-units has been configured to allow a finite number of numerical tag sub-units.

Embodiments of the fifth aspect of the present invention reflect any of the embodiments of the first aspect of the present invention.

The present invention further encompasses a tagged nucleic acid molecule construed according to the method of the fifth aspect of the present invention and its use in a method for multiplex sequencing and/or demultiplexing.

**EXAMPLES**

The example disclosed herein shows that is possible to efficiently multiplex a relatively large number of samples in the MinION™ sequencing platform even with the native ID sequencing chemistry. For this purpose, the genotyping of 30 independent amplicons from the Chikungunya Virus (CHIKV) glycoprotein E1 gene with the MinION™ R9.4 sequencing chemistry and 24-nucleotide barcodes are considered.

The analysis of the CHIKV genome is not feasible as a single molecule, since viral RNA in clinical samples (serum, plasma and urine) exhibit a varying degree of degradation. In respect of technical issues, sequences of CHIKV isolates can be obtained from clinical specimens by amplifying overlapping fragments of the genome for subsequent sequencing (Quick et al. 2017, Nat. Protoc. 12(6):1261-1276).

*Alignment of consensus sequences for synchronization*

Reads were basecalled from .fast5 sequencing files using Albacore, producing fasta/fastq files containing 2D basecalled reads. For each read, a global CS alignment (end-to-end) was performed with the general purpose aligner bowtie2 (Langmead and Salzberg 2012, Nat. Methods 9(4):357-359) set to the end-to-end alignment mode. bowtie2 was configured to make a very loose alignment (-score-min L, -85.0, 0.0 -rdg 0,1 -mp 1,1 -rfg 0,1) in order to deal with the combined effect of a) the sequencing error rate (artificial differences) and, b) the differences between the reference sequence and the sequence actually present in the sample (true differences).

*Variant calling*

Demultiplexed reads where aligned to CHIKV reference genome KP164568 using bwa (Li and Durbin 2009, Bioinformatics 25(14):1754-1760). Following alignment, SAM alignment files were parsed to report all differences between sequencing reads and the reference, with their corresponding quality scores, using an in-house script. The quality-weighted percentage of reads reporting a discordant base at each position was calculated and plotted to identify candidate SNPs.

*Sample collection*

N=30 plasma and serum samples, including replicates, coming from thirteen patients previously diagnosed with CHIKV infection at the Cancer Hospital I, Instituto Nacional de Cancer (INCA), Rio de Janeiro, Brazil, are considered. Twelve of the patients were diagnosed during the 2016 CHIKV outbreak and another unique one was diagnosed on February 2017. Differential laboratory diagnosis was performed by RT-qPCR, as described in (Lanciotti et al. 2007, Emerging Infect. Dis. 13(5):764-767). Serum and plasma samples were stored at -80 °C until viral RNA extraction. Patients signed a Free and Informed Consent Term (TCLE) allowing research to be carried out.

*RNA Extraction, cDNA synthesis and PCR amplicon tailing*

RNA-based amplicons were prepared. Total RNA was extracted with the QIAamp® VIRAL RNA Mini kit (Qiagen, 52904) from 400 $\mu$L of plasma or serum samples coming from individuals previously diagnosed with CHIKV infection by RT-qPCR. Total RNA was retrotranscribed using the High Capacity cDNA Reverse Transcription kit (Applied Biosystems™, 4368814) and a random priming strategy in 20 $\mu$L reactions. PCR amplification of a 1.38 kb segment of the CHIKV genome containing the E1 gene was carried out with tailed primers [3580F + E1_9780F] and [PR2 + E1_11145R]. These tailed primers allow the amplification of East Central South African (ECSA) CHIKV genotype variants by the inclusion of specific primer sequences E1_9780F 5'-TAGCCTAATATGCTGCATCAGAAC-3' and degenerate primer sequences E1_11145R 5'-GGGTARTTGACTATGTGGTCCTTC-3' and subsequent PCR amplicon barcoding by the inclusion of universal adapter sequences 3580F 5'-ACTTGCCTGTCGCTCTATCTTC-3' and PR2 5'-TTTCTGTTGGT-GCTGATATTGC-3'. A 50 $\mu$L PCR reaction was performed with 1 $\mu$L cDNA, 0.2 mM of each dNTP, 0.5 $\mu$mol of each tailed primer, 1X Pfu buffer with MgSO$_4$ and 1.75 U of Pfu DNA Polymerase (Promega, M7741) in a Veriti 96-Well Thermal Cycler (Applied Biosystems). A touchdown thermal profile consisting of 95 °C for 2.5 min, two cycles of 95 °C for 30 secs, 57 °C for 45 secs and 72 °C for 3 min followed by two cycles of 95 °C for 30 secs, 56 °C for 45 secs, 72 °C for 3 min and 32 cycles of 95 °C for 30 secs, 55 °C for 45 secs and 72 °C for 2 min was applied. PCR products were purified with PureLink® Quick Gel Extraction (Invitrogen™, K210012) and quantified in a Qubit® fluorimeter with dsDNA HS Assay Kits (Life Technologies).

*Improved barcodes according to the present invention*

Crude desalted custom 46-mer oligos on a scale of 50 nmol were ordered (GBT Oligos, Argentina). Oligos were processed through normal phase chromatography only removing salts. A total of 64 oligos were designed to contain the 24-mer barcodes sequences plus 22-mer sequences complementary to universal adapters 3580F and PR2. A set of 30 barcodes was selected for the study, leaving the other 34 as negative controls for the experimental estimation of the cross-talk between samples. Improved barcodes where based on the same code parameters used for the 64 24-mer barcode set in (Ezpeleta et al. 2017, Bioinformatics, 33(6):807-813) (q = 8, n = 6, m = 4, u = 4), but without a watermark sequence (or, equivalently, a watermark sequence set to all zeros).

*PCR barcoding amplicons*

RNA-based amplicons were symmetrically barcoded using pairs of custom barcoding primers already tailed with complementary sequences to universal adapters (GBT Oligos, Argentina). Taking into account that 30 barcode libraries would finally pooled, the recommended PCR barcoding protocol was slightly modified and the PCR reaction volume was reduced from 100 to 25 μL. Hence, a PCR reaction was performed with 0.5 nM purified products of the first PCR reaction, 0.2 μmol of each barcode primer and 12.5 μL LongAmpTM Taq 2X Master Mix (New England Biolabs, M0287S) in a Veriti 96-Well Thermal Cycler (Applied Biosystems). A thermal profile of 95 °C for 3 min, followed by 15 cycles of 95 °C for 15 secs, 62 °C for 15 secs, 65 °C for 1:30 min and a final extension step of 3 min at 65 °C was applied. PCR products were purified after carefully mixing with 2x volume of Agencourt AMPure XP beads (Beckman Coulter, Inc A63880) in DNA LoBind tubes (Eppendorf, 022431021) followed by 5 min incubation at room temperature to allow binding. After pelleting on magnet and two washes in 200 μL ethanol (70%) with drying at 37 °C, the pellet was eluted in nuclease-free water (NFW), and pelleted again on a magnet. The eluate was transferred to a fresh DNA LoBind tube. Barcoded RNA-based amplicons from different samples were quantified and combined to achieve the recommended 1 μg DNA input for nanopore sequencing. As a result, 1 μg of pooled barcode libraries in 82.84 μL solution was obtained. Although this volume exceeded the 45 μL solution recommended for the subsequent end-prep reaction, we decided not to concentrate to preserve DNA integrity.

*End-repair and dA tailing*

End-repair and dA tailing were carried out simultaneously with the NEBNext® UltraTMII End-Repair/dA-tailing Modules (New England BioLabs, E7546S) by slightly modifying the protocol to deal with the 82.84 μL of pooled libraries. Following NEB E7546S protocol for a reaction with 50 μL of fragmented DNA, we scaled-up the requirements of buffer and enzyme mix to deal with 82.4 μL. Hence, 11.59 μL of Ultra II End-Prep buffer and 4.9 μL of end repair enzyme mix were added to the 82.84 μL of barcoded amplicons. In addition, 5 μL of a control DNA sequence ('DNA CS') from the Ligation Sequencing Kit 2D (SQK-LSK208) was added as positive control. After incubation at 20 °C for 5 min and 65 °C for 5 min, the end-repaired and dA-tailed DNA library was cleaned-up using 100 μL of Agencourt AMPure XP beads at room temperature, as described above, with elution in 31 μL NFW. Quantification of 1 μL of end-prepped DNA with the Qubit® fluorimeter showed a DNA concentration of 17.2 ng μL$^{-1}$ or, equivalently, 516 ng of fragmented DNA in 30 μL solution, suitable for subsequent sequencing library preparation with the Ligation Sequencing Kit 2D (SQK-LSK208).

*Adapter ligation for the 2D R9.4 sequencing chemistry*

Ligation of adapters was carried out by mixing carefully 10 μL of the double-stranded oligonucleotide Adapter Mix 2D (AMX2D) and 2 μL HP adapters (HPA) in 8 μL NFW and 50 μL of Blunt/TA Ligase Master Mix (New England BioLabs) that were added in that order to 30 μL of the DNA library obtained in the former step. The reaction was incubated at room temperature for 10 min, and 1 μL HP Tether (HPT) was added and incubated for another 10 min at room temperature. The adaptor-ligated, tether-bound library was purified using an equal volume of MyOne C1-beads (Life Technologies) which were washed twice and resuspended in a final volume of 100 μL Bead Binding Buffer (BBB). After 5 min incubation at room temperature to allow binding, beads with library bound were carefully washed 2x with 150 μL BBB buffer. The pelleted beads containing the adapted library were resuspended in 15 μL elution buffer (ELB) and incubated at 37 °C for 10 minutes. After pelleting beads on the magnet, the eluate was transferred to a new DNA LoBind tube and quantified to be used immediately or stored at -20 °C. Quantification of 1 μL of end-prepped DNA with the Qubit® fluorimeter showed a DNA concentration of 10.49 μg μL$^{-1}$ or, equivalently, 157 ng of fragmented DNA in 15 μL solution, suitable for subsequent library loading into the MinION Flow Cell.

*MinION™ Flow Cell preparation and library loading*

Sequencing was performed on MinIONT™ SpotON Flow Cells MK I (R9.4) (Oxford Nanopore Technologies, FLO-SPOTR9). Platform QC was performed to determine the number of active pores and about 1400 active pores were obtained. The flowcell was primed with 800 μL priming buffer (480 μL Oxford Nanopore Running Buffer (RBF) and 520 μL NFW) via the priming port. After 5 min incubation at room temperature, 200 μL priming buffer was added via the priming port. During flowcell preparation, 12 μL of final library was mixed by pipetting with 25.5 μL Library Loading Beads (Oxford Nanopore Technologies, LLB), 35 μL RBF and 2.5 μL NFW. Immediately after priming, 75 μL of the prepared library was loaded onto the flowcell through the SpotON sample port in a dropwise fashion. R9.4 raw data were generated during a sequencing run of 15 hours.

*Sanger sequencing*

Human samples (serum and plasma) from 9 individuals positive for CHIKV qRT-PCR were randomly selected amongst those sampled between week 52 of 2016 and week 5 of 2017 within the epidemic phase in Rio de Janeiro, and those having a high viral load (mostly between 5.5 and 7.0 logs copies/ml). The 1.38 kb fragment of CHIKV genome amplified with primers E1_9780F (5'-TAGCCTAATATGCTGCATCAGAAC-3') and E1_11145R (5'-GGGTARTTGACTATGTGGTCCTTC-3') was sequenced bidirectionally using BigDyeTM v.3.1 (Applied Biosystems), and analyzed in an ABI 3130xl DNA Sequencer (Applied Biosystem, Foster City, CA). Sequencing primers were the same as for RT-PCR amplification and in some cases, an internal primer (5'-GCTCCGCGTCCTTTACC-3'; coordinates 10389-10943) was used to complete 3' sequences.

*SNP calling in CHIKV*

Successfully demultiplexed reads in either ID or 2D mode where used to call variants within the coding region of CHIKV gene E1. Identified SNPs included an A->C mutation at position 632, which was shared by all samples, four sample-specific SNPs at positions 10219, 10204, 10336 and 10392, and a further SNP at position 11112 which corresponded to a degenerate base in the reverse primer (which was expected to contain an equimolar mixture of T and G).

As shown in Figure 1 for 2D and fwd ID reads, these SNPs stand out clearly from background noise. Interestingly, degenerate base of the reverse primer is shown with roughly half the abundance of other SNPs, as expected, meaning coverage is sufficient, in most cases, to not only identify SNPs but also provide a rough quantitative estimate of the abundance. Identified SNPs where consistent across samples belonging to the same patient and with independent Sanger sequencing of nine of the patients. Additionally, no cross-talk was observed between samples, which is consistent with the very low-level of cross-talk reported in Table 1.

Table 1

| Read type | Raw reads | Length-compliant reads | Demultiplexed reads | False positives | Agreement with | | |
|---|---|---|---|---|---|---|---|
| | | | | | 2D | *Template* 1D | *Complement* 1D |
| 2D | 90637 | 47284 | 30501 (64.51%) | 7 (0.02%) | 100% | 99.63% | 99.54% |
| *Template* | 143771 | 72114 | 43179 (59.88%) | 74 (0.17%) | 99.63% | 100% | 99.22% |
| *Complement* | 93554 | 46243 | 16713 (36.14%) | 27 (0.16%) | 99.54% | 99.22% | 100% |

Experimental results show that efficient multiplex 1D sequencing can be performed on the MinION™ sequencing platform with the improved barcodes according to the present invention. 1D sequencing chemistry is less expensive, less involved, more rapid and with higher throughput than the 2D variants. Furthermore, as regards downstream bioinformatic analysis, quite similar results are obtained by means of 2D and *fwd* 1D demultiplexed reads. This is in agreement with the higher throughput of the 1D sequencing chemistry that makes its work at compensating its higher error rate (18 vs. 11%). On the other hand, the extreme error 25% error rate observed in *rev* 1D reads, which has been noted by MinION™ community users, is likely the culprit behind the comparatively poor number of surviving *rev* 1D reads after demultiplexing. On the bright side, however, the false positive rate for *rev* 1D reads is essentially the same as for *fwd* reads, suggesting our filtering criteria are being successful at discarding reads which cannot be called confidently and, even at 25% error rate, extremely low crosstalk is observed.

*Sequences*

| Name | SEQ ID NO. | Sequence |
|---|---|---|
| Primer sequence E1_9780F | 1 | 5'-TAGCCTAATATGCTGCATCAGAAC-3' |
| Primer sequence E1_11145R | 2 | 5'-GGGTARTTGACTATGTGGTCCTTC-3' |
| Adapter sequence 3580F | 3 | 5'-ACTTGCCTGTCGCTCTATCTTC-3' |
| Adapter sequence PR2 | 4 | 5'-TTTCTGTTGGTGCTGATATTGC-3' |
| Internal primer sequence | 5 | 5'-GCTCCGCGTCCTTTACC-3' |

**Claims**

1. A method of tagging a nucleic acid molecule with a predetermined ID number, the method comprising:

   a. attaching to said nucleic acid molecule a nucleic acid tag to form a tagged nucleic acid molecule,

   wherein said nucleic acid tag comprises one or more nucleic acid tag sub-units, each consisting of groups of at least two nucleotides,
   wherein said nucleic acid tag is attributed said ID number by performing the following steps:

   i. converting each nucleotide in said nucleic acid tag sub-units into a number ranging from 0 to 3, thereby creating numerical tag sub-units, wherein the distribution and content of the nucleotides in the nucleic acid tag sub-units has been configured to allow a finite number of numerical tag sub-units,
   ii. attributing to each of said numerical tag sub-units a predetermined numerical tag element, thereby creating a numerical tag,
   iii. linearly decoding said numerical tag, thereby creating said ID number.

2. The method according to claim 1, wherein said numerical tag sub-units are selected by maximizing the minimum Hamming distance of each numerical tag sub-unit relative to the other numerical tag sub-units.

3. The method according to any of the claims 1 and 2, wherein each of said numerical tag elements is a number between 0 and q-1, wherein q is an integer greater than or equal to 8.

4. The method according to any of the claims 1 to 3, wherein said numerical tag is linearly decoded into said ID number by predetermined mathematical operations.

5. The method according to any of the claims 1 to 4, wherein at least one nucleotide in the nucleic acid tag is modified with a detectable label.

6. The method according to any of the claims 1 to 5, wherein the step of attaching to said nucleic acid molecule a nucleic acid tag is performed by any reaction comprising: ligation of a tagged adaptor to said nucleic acid molecule and/or hybridization of a tagged oligonucleotide primer.

7. A method for multiplex sequencing and/or demultiplexing, the method comprising the steps of:

   a. multiplex amplification of tagged nucleic acid molecules obtained according to a method of any of the claims 1 to 6,
   b. pooling and parallel sequencing said plurality of tagged nucleic acid molecules, thereby generating a plurality of sequence reads, and
   c. demultiplexing said plurality of sequence reads, wherein each of said sequence reads is attributed to a sample.

8. The method according to claim 7, wherein step (c) further comprises the steps of

   i. alignment/synchronization of the plurality of sequence reads obtained in step (b) and
   ii. decoding said plurality of sequence reads.

9. The method according to any of the claims 7 and 8, wherein said step of sequencing is performed according to any of the method comprising: single-molecule real-time sequencing, pyrosequencing, sequencing by synthesis, sequencing by ligation, nanopore sequencing, Sanger sequencing, capillary electrophoresis sequencing.

10. The method according to any of the claims 7 to 9, wherein a rate of sequence misassignment of 1 in every 500 reads, preferably less than 1 in every 2500 reads.

11. A tagged nucleic acid molecule construed according to the method of any of the claims 1 to 6.

12. Use of a tagged nucleic acid molecule according to claim 11 in a method for multiplex sequencing and/or demultiplexing.

13. Apparatus configured for multiplex sequencing and/or demultiplexing, the apparatus comprising:

   - tools for designing nucleic acid tags according to any of the claims 1 to 6,
   - tools for pooling and multiplexing a plurality of tagged nucleic acid sequences,
   - a sequence demultiplexer,
   - additional tools for data reproduction and post-sequencing analysis.

14. A method of tagging a nucleic acid molecule with a predetermined ID number, the method comprising:

   a. attaching to said nucleic acid molecule a nucleic acid tag to form a tagged nucleic acid molecule,

   wherein said nucleic acid tag comprises one or more nucleic acid tag sub-units each consisting of groups of at least two nucleotides,
   wherein said nucleic acid tag is obtained from said ID number by performing the following steps:

   i. linearly encoding said ID number, thereby creating a numerical tag, wherein the numerical tag comprises a plurality of numerical tag elements,
   ii. attributing to each of said numerical tag elements a numerical tag sub-unit, wherein the numerical tag sub-unit comprises a plurality of numbers ranging from 0 to 3,
   iii. attributing to each of said numerical tag sub-units a nucleic acid tag sub-unit, thereby creating said nucleic acid tag,

   wherein the distribution and content of the nucleotides in the nucleic acid tag sub-units has been configured to allow a finite number of numerical tag sub-units.

Figure 1

Figure 2

Europäisches
Patentamt

European
Patent Office

Office européen
des brevets

# EUROPEAN SEARCH REPORT

**Application Number**

EP 17 19 7597

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JOAQU\'IN EZPELETA ET AL: "Designing robust watermark barcodes for multiplex long-read sequencing", ARXIV.ORG, CORNELL UNIVERSITY LIBRARY, 201 OLIN LIBRARY CORNELL UNIVERSITY ITHACA, NY 14853, 5 April 2016 (2016-04-05), XP080693521, * pgs. 3-6, Figs. 1, 3 * | 1-14 | INV. G06F19/20 G06N3/12 |
| X | TILO BUSCHMANN ET AL: "Levenshtein error-correcting barcodes for multiplexed DNA sequencing", BMC BIOINFORMATICS, vol. 14, no. 1, 1 January 2013 (2013-01-01), page 272, XP055194297, ISSN: 1471-2105, DOI: 10.1186/1471-2105-14-272 * abstract, Introduction, pgs. 2-3, Table 2 * | 1-14 | |
| X | LEONID V. BYSTRYKH: "Generalized DNA Barcode Design Based on Hamming Codes", PLOS ONE, vol. 7, no. 5, 17 May 2012 (2012-05-17), page e36852, XP055374134, DOI: 10.1371/journal.pone.0036852 * abstract, introduction, pgs. 2, 5, 6 * | 1-14 | TECHNICAL FIELDS SEARCHED (IPC) G06F G06N |
| X | US 2015/087537 A1 (HUBBELL EARL [US]) 26 March 2015 (2015-03-26) * [0047], [0077]-[0104], claims 1, 9 * | 1-14 | |

The present search report has been drawn up for all claims

| Place of search | Date of completion of the search | Examiner |
|---|---|---|
| Munich | 17 April 2018 | Wimmer, Georg |

## ANNEX TO THE EUROPEAN SEARCH REPORT
## ON EUROPEAN PATENT APPLICATION NO.

EP 17 19 7597

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2018

| Patent document cited in search report | Publication date | Patent family member(s) | Publication date |
|---|---|---|---|
| US 2015087537 A1 | 26-03-2015 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HARISMENDY et al.** *Genome Biology,* 2009, vol. 10 (3), R32
- **PARAMESWARAN et al.** *Nucleic Acids Res.,* 2007, vol. 35 (19), e130
- **MEYER et al.** *Nucleic Acids Res.,* 2007, vol. 35 (15), e97
- **BINLADEN et al.** *PLoS ONE,* 2007, vol. 2 (2), e197
- **DOHM et al.** *Nucleic Acids Research,* 2008, vol. 36 (16), e105
- **MINOCHE et al.** *Genome Biology,* 2011, vol. 12, R112
- **TAPIA et al.** *PLoS ONE,* 2015, vol. 10 (10), e0140459
- **KRACHT ; SCHOBER.** *BMC Bioinformatics,* 2015, vol. 16, 50
- **DAVEY ; MACKAY.** *Communications Letters IEEE,* 1998, vol. 2 (6), 165-167
- **EZPELETA et al.** *Bioinformatics,* 2017, vol. 33 (6), 807-813
- **HAMADY et al.** *Nat. Methods,* 2008, vol. 5 (3), 235-237
- **BUSCHMANN ; BYSTRYKH.** *BMC Bioinformatics,* 2013, vol. 14, 272
- **BYSTRYKH.** *PLoS ONE,* 2012, vol. 7 (5), e36852
- **KRISHNAN et al.** *Electronics Letters,* 2011, vol. 47 (4), 236-237
- **BRANTON et al.** Nature. *Biotechnology,* 2008, vol. 26, 1146-1153
- **QUICK et al.** *Nat. Protoc.,* 2017, vol. 12 (6), 1261-1276
- **VAN DER HELM et al.** *Nucleic Acids Res.,* 2017, vol. 45 (8), e61
- **BAYLISS et al.** *Gigascience,* 2017, vol. 6 (3), 1-6
- **LANGMEAD ; SALZBERG.** *Nat. Methods,* 2012, vol. 9 (4), 357-359
- **LI ; DURBIN.** *Bioinformatics,* 2009, vol. 25 (14), 1754-1760
- **LANCIOTTI et al.** *Emerging Infect. Dis.,* 2007, vol. 13 (5), 764-767

SEQUENCE LISTING

<110> Consejo Nacional de Investigaciones Científicas y Tecnicas (CONICET)

<120> METHOD OF TAGGING NUCLEIC ACID SEQUENCES, COMPOSITION AND USE THEREOF

<130> B222-0005EP1

<160> 5

<170> PatentIn version 3.5

<210> 1
<211> 24
<212> DNA
<213> Artificial Sequence

<220>
<223> Primer sequence

<400> 1
tagcctaata tgctgcatca gaac                                                   24

<210> 2
<211> 24
<212> DNA
<213> Artificial Sequence

<220>
<223> Primer sequence

<400> 2
gggtarttga ctatgtggtc cttc                                                   24

<210> 3
<211> 22
<212> DNA
<213> Artificial Sequence

<220>
<223> Adapter sequence

<400> 3
acttgcctgt cgctctatct tc                                                     22

<210> 4
<211> 22
<212> DNA
<213> Artificial Sequence

<220>
<223> Adapter sequence

<400> 4
tttctgttgg tgctgatatt gc                                                     22

<210> 5

```
<211>  17
<212>  DNA
<213>  Artificial Sequence

<220>
<223>  Primer sequence

<400>  5
gctccgcgtc ctttacc                                                    17
```